(19) Europäisches Patentamt
European Patent Office
Office européen des brevets

(11) Publication number: 0 006 633
B1

(12) **EUROPEAN PATENT SPECIFICATION**

(45) Date of publication of patent specification: 03.02.82

(21) Application number: 79102194.2

(22) Date of filing: 29.06.79

(51) Int. Cl.³: C 07 D 513/04,
A 01 N 43/90
//(C07D513/04, 277/00,
235/00)

# (54) 6-(3,5-Dichlorophenyl)perhydroimidazo(5,1-b)thiazole derivatives, antifungal compositions and a method of controlling fungal infections in plants.

(30) Priority: 30.06.78 JP 79442/78
20.09.78 JP 115372/78

(43) Date of publication of application:
09.01.80 Bulletin 80/1

(45) Publication of the grant of the European patent:
03.02.82 Bulletin 82/5

(84) Designated Contracting States:
CH DE FR GB IT NL

(56) References cited:
DE - A - 1 958 183
DE - A - 2 160 912
DE - A1 - 2 604 989

The file contains technical information submitted after the application was filed and not included in this specification.

(73) Proprietor: MITSUBISHI CHEMICAL INDUSTRIES LIMITED
5-2, Marunouchi 2-Chome Chiyoda-Ku
Tokyo 100 (JP)

(72) Inventor: Shigematsu, Taichiro
No. 1810-12, Aihara-machi
Machida-shi, Tokyo (JP)
Inventor: Yoshida, Kenji
No., 3, Sakuradai, Midori-ku
Yokohama-shi, Kanagawa-ken (JP)
Inventor: Nakazawa, Makoto
No. 17-6, 4-chome, Kamitsuruma
Sagamihara-shi, Kanagawa-ken (JP)
Inventor: Kasugai, Hiroshi
No. 4370, Hino-machi, Kohnan-ku
Yokohama-shi, Kanagawa-ken (JP)
Inventor: Tsuda, Masataka
No. 10-2, 2-chome, Ogawa
Machida-shi, Tokyo (JP)

(74) Representative:
Vossius.Vossius.Tauchner.Heunemann.Rauh et al
Siebertstrasse 4 P.O. Box 86 07 67
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 006 633 B1

# 6-(3,5-dichlorophenyl)perhydroimidazo[5,1-b]thiazole derivatives, antifungal compositions and a method of controlling fungal infections in plants

This invention relates to novel 6-(3,5-dichlorophenyl)perhydroimidazo[5,1-b]thiazole-5,7-diones and 6-(3,5-dichlorophenyl)perhydroimidazo-[5,1-b]thiazole-5-thion-7-ones, which are hydantoin derivatives and which show excellent fungicidal activity.

A number of 3-substituted hydantoin-type compounds have been known. In DE-A 2 604 989 1,5-alkylene-3-arylhydantoin derivatives which are useful as herbicides and/or fungicides are disclosed. In particular, 1,5-alkylene-3-(3,5-dichlorophenyl)hydantoin is shown as a fungicide. DE-A 1 958 183 discloses 3-(3,5-dichlorophenyl)hydantoin derivatives which may bear an alkyl group in the 5-position, which may be substituted by a methylmercapto group.

The object of the invention is to provide novel hydantoin derivatives having the formula (I) indicated below, i.e. 6-(3,5-dichlorophenyl)perhydroimidazo[5,1-b]thiazole-5,7-diones or 6-(3,5-dichlorophenyl)perhydroimidazo[5,1-b] thiazole-5-thion-7-ones, which show remarkable fungicidal effects on plant pathogenic fungi.

This invention relates to 6-(3,5-dichlorophenyl)perhydroimidazo[5,1-b]thiazole derivatives of the formula I

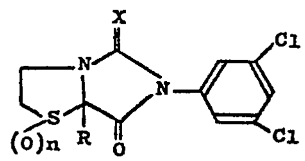

(I)

wherein R represents a hydrogen atom or an alkyl group having 1 to 4 carbon atoms, X represents an oxygen atom or a sulfur atom, and n is zero, 1 or 2. This invention also includes fungicidal compositions comprising as their effective component the above-mentioned 6-(3,5-dichlorophenyl)perhydro-imidazo[5,1-b]thiazole compounds of the formula I and methods of controlling fungal infection in plants by applying the compounds and/or compositions to the plants.

Specific examples of the alkyl group are methyl, ethyl, propyl and butyl. All the compounds of the invention of the formula I are novel and exhibit a powerful fungicidal action against plant diseases, but produce little or no damage to the plants themselves. In addition, these compounds are low in toxicity for humans, animals and fish. The compounds of the invention are extremely effective against various diseases of vegetables, fruit trees and rice, especially grey moulds (Botrytis cinerea, Botrytis allii), sclerotinia rots (Sclerotinia sclerotiorum), apple leaf spot (Alternaria mali), pear black spot (Alternaria kikuchiana), rice blast (Pyricularia oryzae), and rice brown spot (Cochliobolus miyabeanus).

Moreover, the compounds of the invention are highly effective against fungi which are resistant to conventional fungicides (e.g. Benomyl, Thiophanat-methyl, and Polyoxin B).

Among the present compounds in view of the fungicidal activity those compounds of the formula I are preferred, in which X is an oxygen atom, R is a hydrogen atom or an alkyl group with 1 to 3 carbon atoms and n is 0, more preferably the compounds of the formula I in which X is an oxygen atom, R is a hydrogen atom, methyl group or ethyl group and n is 0.

On account of their higher degree of fungicidal activity and ease of synthesis, most preferred are the compounds of the formula I 6-(3,5-dichlorophenyl)perhydroimidazo[5,1-b]thiazole-5,7-dione and 7a-methyl-6-(3,5-dichlorophenyl)perhydroimidazo[5,1-b]thiazole-5,7-dione.

The compounds of the formula I are prepared by intramolecular condensation of thiazolidine-2-carboxylic acid derivatives of the formula II

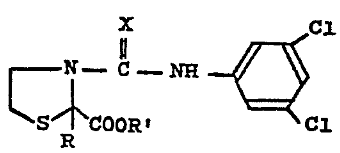

(II)

in which R and X have the meaning given above and R′ represents a hydrogen atom or an alkyl group having 1 to 5 carbon atoms.

The compounds of the formula II can be prepared by reacting a thiazolidine-2-carboxylic acid, which may be substituted in the 2-position by an alkyl group with 1 to 4 carbon atoms or its lower alkyl ester with 3,5-dichlorophenyl-isocyanate or 3,5-dichlorophenylisothiocyanate. This can be expressed by the following reaction scheme:

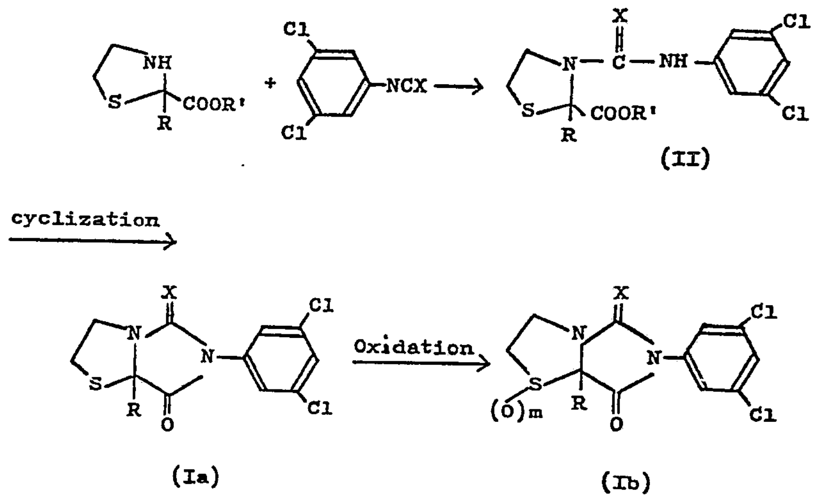


(II)

(Ia) (Ib)

(wherein R and X have the same meanings as defined in the foregoing formula I, R′ represents a hydrogen atom or an alkyl group with 1 to 5 carbon atoms, and m is 1 or 2).

When the lower alkyl esters of a thiazolidine-2-carboxylic acid are used as starting material, the esters are reacted with 3,5-dichlorophenylisocyanate or 3,5-dichlorophenylisothiocyanate in a solvent such as benzene, toluene, ether, dimethylformamide or similar to obtain a 3-(3,5-dichlorophenyl-carbomoyl)thiazolidine-2-carboxylic acid ester or a 3-(3,5-dichlorophenylthiocarbamoyl) thiazolidine-2-carboxylic acid ester (where R′=a lower alkyl group in the formula II).

It may be found that the above reaction continues after formation of the 3-substituted thiazolidine-2-carboxylic acid esters (where in the formula II R′=a lower alkyl group) depending on the reaction conditions, to give the 6-(3,5-dichlorophenyl)perhydroimidazo[5,1-b]thiazole-5,7-diones or the 6-(3,5-dichlorophenyl)perhydroimidazo[5,1-b]thiazole-5-thion-7-ones expressed by the formula Ia. From the thiazolidine-2-carboxylic acids used as starting material 3-(3,5-dichlorophenyl-carbamoyl)thiazolidine-2-carboxylic acids or 3-(3,5-dichlorophenylthiocarbamoyl)thiazolidine-2-carboxylic acids (in the formula II, R′=a hydrogen atom) can be obtained by suspending or dissolving the acids in a mixture of an organic solvent such as benzene, chlorobenzene, ether, or dimethylformamide and water, reacting with 3,5-dichlorophenylisocyanate or 3,5-dichlorophenylisothiocyanate in the presence of an alkali such as caustic soda or caustic potash, and then neutralizing with a mineral acid such as hydrochloric acid or sulfuric acid.

The compounds of the formula II are then cycled to the 6-(3,5-dichlorophenyl)perhydroimidazo-[5,1-b]thiazole-5,7-diones or 6-(3,5-dichlorophenyl) perhydromidazo[5,1-b]thiazole-5-thion-7-ones (Ia). The compounds are generally heated in the presence of a mineral acid such as hydrochloric acid or sulfuric acid or heated in acetic anhydride in the presence of an alkali metal salt of acetic acid such as sodium acetate. In this case, heating is carried out to above about 50°C, preferably to 80—150°C. It is also possible to carry out the reaction in the presence of an alcoholate of an alkali metal such as sodium alcoholate or potassium alcoholate in a lower alkanol such as ethanol or methanol. These compounds are purified by conventional methods such as recrystallization or chromatography. 6-(3,5-dichlorophenyl)perhydroimidazo[5,1-b]thiazole-5,7-dione-1-oxides or -1,1-dioxides or 6-(3,5-dichlorophenyl)perhydromidazo[5,1-b]thiazole-5-thion-7-one-1-oxides or -1,1-dioxides (Ib) can then be obtained by dissolving the compounds (Ia) in a solvent such as methylene chloride and oxidizing them with an oxidizing agent such as m-chloroperbenzoic acid or peracetic acid.

When used as agricultural fungicides, the compounds of the invention may be applied as they are, but are preferably admixed with adjuvants, e.g. solvents, fillers, surface-active agents, e.g. in the form of emulsions, wettable powders or dusts.

Solvents suitable as adjuvants for the fungicides of the invention are, for example, water, alcohols (such as methyl alcohol, ethyl alcohol or ethylene glycol), ketones (such as acetone, methyl ethyl ketone or cyclohexanone), ethers (such as ethyl ether, dioxane or cellosolves), alipatic hydrocarbons (such as kerosene, lamp oil or fuel oil), aromatic hydrocarbons (such as benzene, toluene, xylene, solvent naphtha or methylnaphthalene), halogenated hydrocarbons (such as dichloroethane, trichlorobenzene or carbon tetrachloride), acid amides (such as dimethylformamide), esters (such as ethyl acetate, butyl acetate or glycerine esters of aliphatic acids) and nitriles (such as acetonitrile). These solvents may be used singly or in combinations of two or more.

Suitable fillers are, for example, mineral powders including clays such as kaolin or bentonite, talcs such as talc or pyrophyllite, mineral powders such as diatomaceous earth, white carbon ($SIO_2$), plant powders such as soy bean powder or CMC powder. These may be used singly or in combinations of two or more.

Surface-active agents may be used as spreaders, dispersants, emulsifiers or penetrants. Examples

of surface-active agents include nonionic active agents (such as polyoxyethylene alkylaryl ethers or polyoxyethylene sorbitan monolaurate), cationic active agents (such as alkyldimethylbenzylammonium chlorides or alkyl-pyridinium chlorides), anionic active agents (such as alkylbenzene sulfonates, lignin sulfonates, higher alcohol sulfates) and amphoteric surfactants (such as alkyldimethyl-betaines or dodecylaminoethylglycine). These surface-active agents may be used singly or in combination, depending on the envisaged end-purpose.

When the agricultural fungicide is used in the form of an emulsion, a formulated concentrate obtained by mixing 10—50 parts of the compound of the invention, 10—80 parts of a solvent and 3—20 parts of a surface-active agent is diluted with water to a predetermined concentration and applied by conventional methods such as spraying.

When used in the form of a wettable powder, 5—80 parts of the compound of the invention, 10—90 parts of a filler, and 1—20 parts of a surface-active agent are mixed in suitable proportions and the mixture is then diluted with water as in the case of the emulsion for subsequent use.

In the case of a dust, it is usual to uniformly mix 1—5 parts of the compound of the invention with 95—99 parts of a filler such as kaolin, bentonite or talc and apply the mixture.

The fungicidal compositions according to the invention may contain other active components which do not impede the fungicidal effect of the inventive compounds, e.g. other fungicides, insecticides or miticides.

For the treatment of foliage, the fungicidal compositions of the invention are used in an amount of 50—500 l of a solution containing 250—1500 ppm of the active ingredient per 10 ares.

The present invention will be described in more detail by way of Preparatory Examples of the compounds of the invention, Preparation Examples of the fungicidal compositions comprising the present compounds, and Experimental Controlling Examples using the fungicides, which should not be construed as limiting the invention thereto. If not otherwise stated, parts are by weight.

Preparatory Example 1

56.8 g of cysteamine hydrochloride and 50.6 g of triethylamine were dissolved in 500 ml of ethanol, to which was added 148.1 g of an aqueous 25% glyoxylic acid solution. The mixture was refluxed for 1.5 hours, cooled and filtered, thereby giving 41.6 g (yield 62.5%) of thiazolidine-2-carboxylic acid. The melting point was 187—189°C (dec.).

6.66 g of thiazolidine-2-carboxylic acid thus obtained and 2.0 g of caustic soda were dissolved in 100 ml of water, to which was added a solution of 9.40 g of 3,5-dichlorophenylisocyanate in 50 ml of chlorobenzene, followed by stirring at room temperature for 2 hours. The reaction mixture was then extracted with ether and the water layer neutralized with concentrated hydrochloric acid. The resulting crystals were separated, washed with water and dried to give 14.4 g (yield 89.5%) of 3-(3,5-dichlorophenylcarbamoyl)thiazolidine-2-carboxylic acid. The melting point was found to be 175.5—177.0°C.

Elementary Analysis: Found (calculated in brackets) C 40.88% (41.14%), H 3.11% (3.14%), N 8.86% (8.72%), Cl 22.24% (22.08%).

A mixture of 13.0 g of 3-(3,5-dichlorophenylcarbamoyl)thiazolidine-2-carboxylic acid thus obtained and 50 ml of concentrated hydrochloric acid was stirred at 120°C for 2 hours. The reaction mixture was then cooled and filtered to separate the precipitate, followed by recrystallization from a mixture of ethyl acetate and n-hexane to give 10.7 g (yield 86.9%) of 6-(3,5-dichlorophenyl)perhydroimidazo[5,1-b]thiazole-5,7-dione (Compound No. 1). Its melting point and elementary analysis are given in Table I.

Preparatory Example 2

A mixture of 20.0 g of the thiazolidine-2-carboxylic acid obtained in Preparatory Example 1 and 200 ml of a 20% solution of hydrogen chloride in ethanol was refluxed for 2 hours. The solvent was distilled off under reduced pressure, followed by adding water and neutralizing with an aqueous saturated sodium bicarbonate. After extraction with ethyl acetate, the extract was vacuum distilled to give 19.1 g (yield 79.8%) of thiazolidine-2-carboxylic acid ethyl ester. The boiling point was found to be 104—105°C/5.5 Torr.

A mixture of 1.61 g of thiazolidine-2-carboxylic acid ethyl ester thus obtained, 2.04 g of 3,5-dichlorophenylisothiocyanate and 20 ml of toluene was heated at 90°C for 1 hour. The reaction mixture was then cooled and the precipitate separated by filtration to give 3.11 g (yield 85.1%) of 3-(3,5-dichlorophenylthiocarbamoyl) thiazolidine-2-carboxylic acid ethyl ester. The melting point was 170.5—171.5°C and the results of elementary analysis were as follows: Found (calculated in brackets) C 42.68% (42.74%), H 3.93% (3.86%), N 7.70% (7.67%), Cl 19.32% (19.41%).

A mixture of 2.19 g of the obtained 3-(3,5-dichlorophenylthiocarbamoyl)thiazolidine-2-carboxylic acid ethyl ester and 20 ml of concentrated hydrochloric acid was stirred at 120°C for 1.5 hours. After the reaction mixture was cooled the precipitate was separated, followed by recrystallization from a mixture of ethyl acetate and n-hexane to give 1.43 g (yield 74.7%) of 6-3,5-dichlorophenyl)perhydroimidazo[5,1-b]thiazole-5-thion-7-one (Compound No. 2). The melting point and the elementary analysis are given in Table I.

Preparatory Example 3

A mixture of 36.6 g of cysteamine hydrochloride, 32.6 g of triethylamine, 32.9 g of methyl pyruvate and 500 ml of ethanol was refluxed for 2 hours. The reaction mixture was concentrated under reduced pressure to give a residue which, after addition of water, was extracted with ethyl acetate. The extract was vacuum distilled to give 36.6 g (yield 70.4%) of 2-methylthiazolidine-2-carboxylic acid methyl ester with a boiling point of 76°C/2 Torr.

3.22 g of the thus obtained 2-methylthiazolidine-2-carboxylic acid methyl ester was added to a solution of 0.80 g of caustic soda in 20 ml of water and stirred at room temperature to give an aqueous solution of sodium 2-methylthiazolidine-2-carboxylate. To the solution was added 3.76 g of 3 5-dichlorophenylisocyanate in 20 ml of chlorobenzene, followed by stirring at room temperature for 1.5 hours. The reaction mixture was then extracted with ether and the water layer neutralized with concentrated hydrochloric acid. The resulting crystals were separated, washed with water and dried to give 5.22 g (yield 77.9%) of 2-methyl-3-(3,5-dichlorophenylcarbamoyl)thiazolidine-2-carboxylic acid with a melting point of 164—166°C. The results of the elementary analysis were as follows: Found (calculated in brackets. C 43.21% (43.00%), H 3.58% (3.61%), N 8.28% (8.36%), Cl 21.28% (21.15%)

5.0 g of the thus obtained 2-methyl-3-(3,5-dichlorophenylcarbamoyl)thiazolidine-2-carboxylic acid and 5 mg of sodium acetate were added to 30 ml of acetic anhydride and heated at 90°C for 2 hours. The reaction mixture was concentrated under reduced pressure and water added. The resulting crystals were recrystallized from a mixture of ethyl acetate and n-hexane to give 3.92 g of 7a-methyl-6-(3,5-dichlorophenyl)perhydroimidazo[5,1-b]thiazole-5,7-dione (Compound No. 3). The results of the elementary analysis and the melting point are given in Table I.

Preparatory Example 4

A mixture of 1.61 g of the 2-methylthiazolidine-2-carboxylic acid methyl ester obtained in Preparatory Example 3, and 2.4 g of 3,5-dichlorophenylisothiocyanate was heated at 90°C for 2 hours. The reaction mixture was then concentrated under reduced pressure, followed by recrystallization from a mixture of toluene and n-hexane to give 2.50 g (yield 75.0%) of a 7a-methyl-6-(3,5-dichlorophenyl)perhydroimidazo[5,1-b]thiazole-5-thion-7-one (Compound No. 4) with a melting point of 160—161°C. The results of the elementary analysis and the melting point are given in Table I.

Preparatory Example 5

1.52 g of the compound No. 1 obtained in Preparatory Example 1 was dissolved in 10 ml of methylene chloride, to which was added, under ice cooling, 1.04 g of m-chloroperbenzoic acid in 12.5 g of methylene chloride, followed by heating under reflux for 1 hour. Thereafter, 1.04 g of m-chloroperbenzoic acid in 12.5 ml of methylene chloride was again added to the reaction system, which was refluxed for a further 1 hour to complete the reaction. After the reaction solution had been cooled and washed with dilute caustic soda, the solution was concentrated to give a residue which was chromatographed on a silica gel column with ethyl acetate to give 0.50 g (yield 29.8%) of 6-(3,5-dichlorophenyl)perhydroimidazo[5,1-b]thiazole-5,7-dione-1,1-dioxide (Compound No. 5). The results of the elementary analysis and the melting point are given in Table I.

Preparatory Example 6

The same procedure as in Preparatory Example 5 was repeated, however this time using 1.52 g of the compound No. 3 obtained in Preparatory Example 3 instead of the compound No. 1. After completion of the reaction the reaction mixture was washed with a dilute caustic soda, concentrated, and then recrystallized from a mixture of ethyl acetate and n-hexane to give 1.18 g (yield 67.6%) of a 7a-methyl-6-(3,5-dichlorophenyl)perhydroimidazo[5,1-b]thiazole-5,7-dione-1,1-dioxide (Compound No. 6). The results of the elementary analysis and the melting point are given in Table I.

Preparatory Example 7

11.4 g of cysteamine hydrochloride and 10.01 g of triethylamine were dissolved in 100 ml of ethanol, to which was added 10.2 g of 2-keto-butyric acid. The mixture was refluxed for 30 minutes, cooled and then filtered, thereby giving 10.6 g (yield 65.7% of 2-ethyl-thiazolidine-2-carboxylic acid with a melting point of 235—238°C (dec.).

10.0 g of the 2-ethyl-thiazolidine-2-carboxylic acid thus obtained was dissolved in 200 ml of 20% solution of hydrogen chloride in methanol. After refluxing for 30 minutes, the reaction mixture was concentrated under reduced pressure to give a residue, to which water was added, and the reaction mixture was then neutralized with aqueous saturated sodium bicarbonate. After extracting with ethyl acetate, the extract was chromatographed on a silica gel column to give 2.0 g (yield 18.4%) of 2-ethyl-thiazolidine-2-carboxylic acid methyl ester with a refractive index $n_D^{26}$=1.501.

A mixture of 0.62 g of the 2-ethyl thiazolidine-2-carboxylic acid methyl ester thus obtained, 0.66 g of 3,5-dichlorophenylisocyanate and 10 ml of toluene was stirred at room temperature for 1 hour. The precipitates were then filtered off to give 1.00 g (yield 77.8%) of 2-ethyl-3-(3,5-dichlorophenyl-

carbamoyl)thiazolidine-2-carboxylic acid methyl ester with a melting point of 187—188°C. The results of the elémentary analysis are as follows: Found (calculated in brackets) C 46.48% (46.29%), H 4.35% (4.44%), N 7.50% (7.71%), Cl 19.86% (19.52%).

0.70 g of 2-ethyl-3-(3,5-dichlorophenylcarbamoyl)thiazolidine-2-carboxylic acid methyl ester was added to 20 ml of 0.1 N solution of sodium methylate in methanol, and heated at 50°C for 20 minutes. The reaction mixture was cooled and filtered. The resulting filtrate was 0.16 g (yield 25%) of 7a-ethyl-6-(3,5-dichlorophenyl)perhydroimidazo[5,1-b]thiazole-5,7-dione (Compound No. 7). The results of the elementary analysis and the melting point are given in Table I.

Preparatory Example 8

The same procedure as in Preparatory Example 7 was repeated, however this time using 2-keto-valeric acid instead of the 2-keto-butyric acid. As a result, the compounds obtained corresponding to the starting material were as follows:

2-n-propyl thiazolidine-2-carboxylic acid
m.p. 222—223°C (dec.)

2-n-propyl thiazolidine-2-carboxylic acid methyl ester
m.p. 39—40°C

2-n-propyl-3-(3,5-dichlorophenylcarbamoyl)thiazolidine-2-carboxylic acid methyl ester
m.p. 187—189°C
Elementary analysis: Found (calculated in brackets)
C 47.36% (47.75%), H 4.95% (4.81%), N 7.61% (7.42%), Cl 18.53% (18.79%)

7a-n-propyl-6-(3,5-dichlorophenyl)perhydroimidazo[5,1-b]thiazole-5,7-dione (Compound No. 8). The results of the elementary analysis and the melting point are given in Table I.

Preparatory Example 9

A mixture of 0.33 g of 2-ethyl-thiazolidine-2-carboxylic acid methyl ester obtained in the Preparatory Example 7, 0.38 g of 3,5-dichlorophenylisothiocyanate and 5 ml of toluene was heated at 90°C for 2 hours and then concentrated to give a residue which was then chromographed on a silica gel column to give 0.15 g of 7a-ethyl-6-(3,5-dichlorophenyl)perhydroimidazo[5,1-b]thiazole-5-thion-7-one (Compound No. 9. The results of the elementary analysis and the melting point are given in Table I.

Preparatory Example 10

The same procedure as in Preparatory Example 9 was repeated, however this time using 3-n-propylthiazolidine-2-carboxylic acid methyl ester obtained in Preparatory Example 8 instead of 2-ethyl-thiazolidine-2-carboxylic acid methyl ester, to give 7a-n-propyl-6-(3,5-dichlorophenyl)perhydro-imidazo[5,1-b]thiazole-5-thion-7-one (Compound No. 10). The results of the elementary analysis and the melting point are given in Table I.

TABLE I

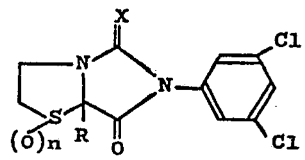

| Compound No. | Substituent R | Substituent X | Substituent n | Melting Point (°C) | Elementary Analysis (Found) (Calculated) C (%) | H (%) | N (%) | Cl (%) |
|---|---|---|---|---|---|---|---|---|
| 1 | H | O | 0 | 141 –142 | 43.40 | 2.73 | 9.18 | 22.25 |
| | | | | | 43.58 | 2.66 | 9.24 | 23.39 |
| 2 | H | S | 0 | 149.5–150.5 | 41.33 | 2.59 | 8.60 | 22.51 |
| | | | | | 41.39 | 2.53 | 8.78 | 22.21 |
| 3 | $CH_3$ | O | 0 | 110 –112 | 45.56 | 3.09 | 8.72 | 22.20 |
| | | | | | 45.44 | 3.18 | 8.83 | 22.35 |
| 4 | $CH_3$ | S | 0 | 160 –161 | 43.36 | 2.95 | 8.36 | 21.41 |
| | | | | | 43.25 | 3.02 | 8.41 | 21.28 |
| 5 | H | O | 2 | 176 –178 | 40.01 | 2.36 | 8.27 | 21.35 |
| | | | | | 39.42 | 2.41 | 8.36 | 21.16 |
| 6 | $CH_3$ | O | 2 | 190 –191 | 41.35 | 2.95 | 7.89 | 20.13 |
| | | | | | 41.28 | 2.89 | 8.02 | 20.31 |
| 7 | $C_2H_5$ | O | 0 | 138 –139 | 46.99 | 3.78 | 8.22 | 21.41 |
| | | | | | 47.14 | 3.65 | 8.46 | 21.41 |
| 8 | n-$C_3H_7$ | O | 0 | 145.5–146.5 | 48.34 | 4.08 | 7.83 | 20.26 |
| | | | | | 48.70 | 4.09 | 8.11 | 20.54 |
| 9 | $C_2H_5$ | S | 0 | 139 –140 | 44.70 | 3.55 | 8.16 | 20.12 |
| | | | | | 44.96 | 3.48 | 8.07 | 20.42 |
| 10 | n-$C_3H_7$ | S | 0 | 153 –153.5 | 46.81 | 3.90 | 7.91 | 19.78 |
| | | | | | 46.54 | 3.91 | 7.75 | 19.62 |

Preparation Example 1

50 parts of Compound No. 1, 45 parts talc, and 5 parts Sorpol 8070 (trade mark, surface-active agent composed principally of higher alcohol sulfate) were uniformly milled and mixed to give a fungicidal composition in the form of a wettable powder.

Preparation Example 2

40 parts of Compound No. 3, 10 parts white carbon, 47 parts diatomaceous earth, and 3 parts Sorpol 5039 (trade mark, surface-active agent composed principally of polyoxyethylene alkyl aryl ether sulfonate) were uniformly milled and mixed to give a fungicidal composition in the form of a wettable powder.

Preparation Example 3

30 parts of Compound No. 2, 15 parts Sorpol 3005X (trade mark, a mixture of a nonionic surface-active agent and an anionic surface-active agent), 25 parts xylene and 30 parts dimethylformamide were mixed and dissolved to give a fungicidal composition in the form of an emulsion.

Preparation Example 4

2 parts of Compound No. 4 and 98 parts N,N-kaolin clay (manufactured by Tsuchiya Kaolin Co., Ltd.) were mixed and pulverized to give a fungicidal composition in the form of a dust.

Test Example 1

Preventive test against Cucumber Grey Mould disease.

A number of cucumber plants (of the variety Satsukimidori) were each grown in a 15 cmφ plastic pot to the first leaf stage. The plants were sprayed with 10 ml/pot of aqueous diluted suspensions of various types of wettable powders (including the wettable powder containing as its active ingredient Compound No. 1 obtained in the Preparation Example 1, wettable powders containing as active ingredients Compounds No. 2—10 obtained as in Preparation Example 1, and a wettable powder as a reference compound containing as its active ingredient tetrachloroisophthalonitrile, obtained as in Preparation Example 1). After air-drying for 5 hours, the plants were inoculated with the mycelium of Botrytis cinerea.

The cucumbers were kept in a humid chamber at 23°C for 4 days after inoculation and then examined with respect to their disease index. The examination was conducted as follows: The leaves were checked to determine a rate of lesion area and classified into six groups as indicated by indices 0, 1, 2, 3, 4, 5 based on the determined rate, and the numbers of leaves $n_0$, $n_1$, $n_2$, $n_3$, $n_4$ and $n_5$ corresponding to the indices, respectively, were also determined to calculate the disease index from the following equation. The results are shown in Table II below.

| Disease Index | Rate of Lesion Area |
|---|---|
| 0 | no lesion |
| 1 | lesion area of 1/5 times total leaf area |
| 2 | ,, 2/5 ,, |
| 3 | ,, 3/5 ,, |
| 4 | ,, 4/5 ,, |
| 5 | ,, above 4/5 ,, |

Disease severity index =

$$\frac{0 \times n_0 + 1 \times n_1 + 2 \times n_2 + 3 \times n_3 + 4 \times n_4 + 5 \times n_5}{n}$$

(where n is the total number of checked leaves)

The preventive value was calculated from the following equation

Preventive Value (%) =

$$\frac{\text{(A) — Disease severity index treated}}{\text{Disease severity index untreated (A)}} \times 100$$

TABLE II

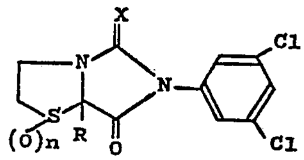

| Compound No. | Substituent | | | Concentration (ppm) | Preventive Value (%) |
|---|---|---|---|---|---|
| | R | X | n | | |
| 1 | H | O | 0 | 500 | 100 |
| | | | | 250 | 100 |
| | | | | 125 | 100 |
| | | | | 62.5 | 100 |
| 2 | H | S | 0 | 500 | 92 |
| 3 | $CH_3$ | O | 0 | 500 | 100 |
| | | | | 250 | 100 |
| | | | | 125 | 100 |
| | | | | 62.5 | 100 |
| 4 | $CH_3$ | S | 0 | 500 | 93 |
| 5 | H | O | 2 | 500 | 91 |
| 6 | $CH_3$ | O | 2 | 500 | 90 |
| 7 | $C_2H_5$ | O | 0 | 500 | 100 |
| 8 | n-$C_3H_7$ | O | 0 | 500 | 97 |
| 9 | $C_2H_5$ | S | 0 | 500 | 96 |
| 10 | n-$C_3H_7$ | S | 0 | 500 | 94 |
| Reference * | | | | 500 | 65 |
| Control | | | | — | 0 |

* reference compound: Tetrachloroisophthalonitrile.

Test Example 2

Preventive test against rice brown spot disease

Rice plants (of the variety Kinmaze) were each grown in 10 cm$\phi$ porcelain pots until 3—4 leaves had formed. The plants were sprayed with 10 ml/pot of an aqueous diluted suspension of various types of wettable powders of the chemicals and the reference compound [bis(dimethyl-thiocarbamoyl)disulfide]. The wettable powders were prepared along the lines of test example 1.

After air-drying for 5 hours, the plants were inoculated with a spore suspension of Cochliobolus miyabeanus. These plants were kept in a humid chamber at 27°C for 48 hours after inoculation, and the number of lesions were counted and the preventive value calculated according to the following equation.

Preventive value (%) =

$$\frac{\text{(A) — number of lesions treated}}{\text{number of lesions untreated (A)}} \times 100$$

The results are given in Table III.

TABLE III

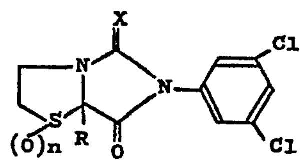

| Compound No. | Substituent | | | Concentration (ppm) | Preventive Value (%) |
|---|---|---|---|---|---|
| | R | X | n | | |
| 1 | H | O | 0 | 500 | 95.1 |
| 2 | H | S | 0 | 500 | 88.4 |
| 3 | $CH_3$ | O | 0 | 500 | 96.2 |
| 4 | $CH_3$ | S | 0 | 500 | 85.7 |
| 5 | H | O | 2 | 500 | 89.6 |
| 6 | $CH_3$ | O | 2 | 500 | 90.6 |
| 7 | $C_2H_5$ | O | 0 | 500 | 90.8 |
| 8 | n-$C_3H_7$ | O | 0 | 500 | 85.4 |
| 9 | $C_2H_5$ | S | 0 | 500 | 82.9 |
| 10 | n-$C_3H_7$ | S | 0 | 500 | 83.2 |
| Reference | * | | | 500 | 83.3 |
| Control | | | | — | 0 |

* reference compound Bis (dimethyl-thiocarbamoyl)disulfide.

Test Example 3

Preventive test against kidney bean stem rot

Kidney bean plants (of the variety Kintoki) were each grown in 15cmø plastic pots to the *trifoliate stage.* The plants were sprayed with 10 ml/pot of an aqueous diluted suspension of wettable powders of the chemicals and the reference compound.

The wettable powders were prepared as in example 1. After air-drying for 5 hours, the plants were inoculated with the mycelium of Sclerotinia sclerotiorum and kept in a humid chamber at 25°C for 4 days. The preventive value was then calculated according to the equation given in test example 1. The results are shown in Table IV.

TABLE IV

| Compound No. | Concentration (ppm) | Preventive Value (%) |
|---|---|---|
| No. 1 | 200 | 100 |
| | 100 | 98.2 |
| | 50 | 95.3 |
| No. 2 | 200 | 100 |
| | 100 | 96.5 |
| | 50 | 93.8 |
| Reference.* | 500 | 70.5 |
| Control | – | 0 |

* Reference compound 2,6-dichloro-4-nitroaniline

Antifungal Test

Each chemical was mixed with a PDA medium in a sufficient amount to obtain the pre-determined concentration level and solidified in a 9 cmϕ Petri dish. Various plant disease fungi were then inoculated onto the middle of the mixed agar plate and cultured at 25°C for 4 days.

The minimum inhibitory concentration (MIC) means that at which the development of the inoculated fungus could not be discerned with the naked eye.

The results are given in Table V:

TABLE V

MIC Concentration (ppm)

| Compd. No. / Fungi Tested | No. 1 | No. 3 | Reference.** |
|---|---|---|---|
| Botrytis cinerea (strain I) | 3.1 | 1.6 | 0.4 |
| Botrytis cinerea (strain II*) | 3.1 | 0.8 | >100 |
| Sclerotinia sclerotiorum | 1.6 | 1.6 | |
| Alternaria mali | 3.1 | 3.1 | >100 |
| Alternaria kikuchiana | 3.1 | 3.1 | >100 |
| Botrytis allii | 6.2 | 3.1 | |

* Botrytis cinerea (strain II) is the strain resistant to benzoimidazole-type fungicide e.g. Benomyl and Thiophanat-methyl.

** Reference compound is Benomyl.

**Claims**

1. 6-(3,5-dichlorophenyl)perhydroimidazo[5,1-b]thiazole derivatives having the formula I

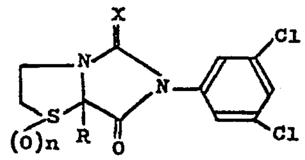

(I)

wherein R represents a hydrogen atom or an alkyl group having 1 to 4 carbon atoms, X represents an oxygen atom or a sulfur atom and n represents zero, 1 or 2.

2. 6-(3,5-dichlorophenyl)perhydroimidazo[5,1-b]thiazole derivatives according to claim 1, wherein n represents zero.

3. 6-(3,5-dichlorophenyl)perhydroimidazo[5,1-b]thiazole derivatives according to claim 2, wherein X represents an oxygen atom.

4. 6-(3,5-dichlorophenyl)perhydroimidazo[5,1-b]thiazole-5,7-dione.

5. 7a-Methyl-6-(3,5-dichlorophenyl)perhydroimidazo[5,1-b]-thiazole-5,7-dione.

6. Antifungal compositions containing an antifungal-effective amount of a 6-(3,5-dichlorophenyl)perhydroimidazo[5,1-b]thiazole derivative according to claim 1.

7. Antifungal compositions containing an antifungal-effective amount of a 6-(3,5-dichlorophenyl)perhydroimidazo[5,1-b]thiazole derivative according to claim 3.

8. Antifungal compositions according to claim 7, wherein R represents a hydrogen atom or methyl group.

9. Method of controlling fungal infections in plants which comprises applying to said plants an antifungal-effective amount of a 6-(3,5-dichlorophenyl)perhydroimidazo[5,1-b]thiazole derivative according to claim 1.

10. Method according to claim 9, wherein n represents zero and X represents an oxygen atom and R represents a hydrogen atom or methyl group.

**Revendications**

1. Dérivés du 6-(3,5-dichlorophényl)-perhydroimidazo[5,1-b]thiazole répondant à la formule I:

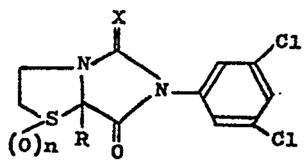

(I)

dans laquelle R représente un atome d'hydrogène ou un groupe alkyle contenant de 1 à 4 atomes de carbone, X représente un atome d'oxygène ou un atome de soufre et n est égal à 0, 1 ou 2.

2. Dérivés du 6-(3,5-dichlorophényl)-perhydroimidazo[5,1-b]thiazole selon la revendication 1, dans lesquels n est égal à 0.

3. Dérivés du 6-(3,5-dichlorophényl)-perhydroimidazo[5,1-b]thiazole selon la revendication 2, dans lesquels X représente un atome d'oxygène.

4. La 6-(3,5-dichlorophényl)-perhydroimidazo[5,1-b]thiazole-5,7-dione.

5. La 7a-méthyl-6-(3,5-dichlorophényl)-perhydroimidazo[5,1-b]thiazole-5,7-dione.

6. Compositions antifongiques contenant une proportion antifongique efficace d'un dérivé de 6-(3,5-dichlorophényl)-perhydroimidazo[5,1-b]thiazole selon la revendication 1.

7. Compositions antifongiques contenant une quantité antifongique efficace d'un dérivé de 6-(3,5-dichlorophényl)-perhydroimidazo[5,1-b]thiazole selon la revendication 3.

8. Compositions antifongiques selon la revendication 7 dans lesquelles R représente un atome d'hydrogène ou un groupe méthyle.

9. Procédé pour combattre les infections fongiques des végétaux, qui consiste à appliquer à ces végétaux une quantité antifongique efficace d'un dérivé de 6-(3,5-dichlorophényl)-perhydroimidazo[5,1-b]thiazole selon la revendication 1.

10. Procédé selon la revendication 9, dans lequel n est égal 0 et X représente un atome d'oxygène et R représente un atome d'hydrogène ou un groupe méthyle.

**Patentansprüche**

1. 6-(3,5-Dichlorphenyl)-perhydroimidazo[5,1-b]thiazol-Derivate der Formel I

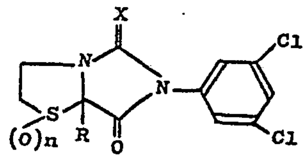

(I)

in der R ein Wasserstoffatom oder einen Alkylrest mit 1 bis 4 Kohlenstoffatomen und X ein Sauerstoffatom oder ein Schwefelatom bedeutet und n den Wert 0, 1 oder 2 hat.

2. 6-(3,5-Dichlorphenyl)-perhydroimidazo[5,1-b]thiazol-Derivate nach Anspruch 1, wobei n den Wert 0 hat.

3. 6-(3,5-Dichlorphenyl)-perhydroimidazo[5,1-b]thiazol-Derivate nach Anspruch 2, wobei X ein Sauerstoffatom bedeutet.

4. 6-(3,5-Dichlorphenyl)-perhydroimidazo[5,1-b]thiazol-5,7-dion.

5. 7a-Methyl-6-(3,5-dichlorphenyl)-perhydroimidazo-[5,1-b]thiazol-5,7-dion.

6. Fungizide Mittel, enthaltend eine fungizid wirksame Menge eines 6-(3,5-Dichlorphenyl)-perhydroimidazo[5,1-b]thiazol-Derivats nach Anspruch 1.

7. Fungizide Mittel, enthaltend eine fungizid-wirksame Menge eines 6-(3,5-Dichlorphenyl)-perhydroimidazo[5,1-b]thiazol-Derivats nach Anspruch 3.

8. Fungizide Mittel nach Anspruch 7, wobei R ein Wasserstoffatom oder eine Methylgruppe bedeutet.

9. Verfahren zur Bekämpfung von Pilzinfektionen bei Pflanzen, dadurch gekennzeichnet, daß man auf die Pflanzen eine fungizid wirksame Menge eines 6-(3,5-Dichlorphenyl)-perhydroimidazo[5,1-b]thiazol-Derivats nach Anspruch 1 aufbringt.

10. Verfahren nach Anspruch 9, wobei n den Wert 0 hat und X ein Sauerstoffatom und R ein Wasserstoffatom oder eine Methylgruppe bedeutet.